# EUROPEAN PATENT SPECIFICATION

(11) **EP 4 294 474 B1**
(45) Date of publication and mention of the grant of the patent: **11.03.2026**
(21) Application number: 22701427.1
(22) Date of filing: 20.01.2022
(51) Int. Cl.: A61M 1/00

(54) **SYSTEMS FOR DETECTING FLUID CONTAMINATION IN A WOUND THERAPY SYSTEM**
SYSTEME ZUR ERKENNUNG VON FLÜSSIGKEITSKONTAMINATIONEN IN EINEM WUNDTHERAPIESYSTEM
SYSTÈMES DE DÉTECTION D'UNE CONTAMINATION PAR UN FLUIDE DANS UN SYSTÈME DE TRAITEMENT DES PLAIES

(30) Priority: 18.02.2021 US 202163150992 P
(43) Date of publication of application: 27.12.2023
(73) Proprietor: KCI Manufacturing Unlimited Company, Athlone, Co. Westmeath, N37XF22 (IE)
(72) Inventor: HOWARD, Robert, Bracknell Berkshire RG12 8HT (GB); PRATT, Benjamin A., Bracknell Berkshire RG12 8HT (GB); INGRAM, Shannon C., San Antonio, Texas 78265 (US)
(74) Representative: Simmons & Simmons
(86) International application number: PCT/IB2022/050468
(87) International publication number: WO 2022/175763

(56) References cited:
- JP-A- H0 875 513
- US-A1- 2011 276 016
- US-A1- 2018 140 466
- US-A1- 2021 038 775

## Description

### BACKGROUND

The present disclosure relates generally to negative pressure wound therapy (NPWT) devices and more particularly monitoring systems for NPWT devices. It would be desirable to provide a NPWT device which detects fluid contamination.
US2021/038775 discloses a negative pressure wound treatment system utilising a piezo-electric pump which uses electrical signals to determine pump status.
JPH0875513 discloses a fluidic flowmeter including a target disposed in the downstream of a flowmeter section which shunts fluid to generate pressure oscillation which can be measured. US2011/0276016 discloses a wound dressing with an aspiration port for equalising pressure and a liquid sensor for sensing liquid within the wound dressing.

US2018/140466 discloses a pump arrangement for topical negative pressure therapy, comprising two pump assemblies arranged in parallel to another.

### SUMMARY

The invention is defined by the independent claim. A selection of optional features of the invention is set out in the dependent claims.

### BRIEF DESCRIPTION OF THE DRAWINGS

FIG. 1A is a block diagram of a therapy system for detecting fluid contamination using a piezo-electric pump, according to some embodiments.
FIG. 1B is a block diagram of the therapy system of FIG. 1 A including a one-way valve, according to some embodiments.
FIG. 2 is a block diagram of a control system for the therapy systems of FIGS. 1 A-1B, according to some embodiments.
FIG. 3 is a block diagram of a control system for the therapy systems of FIGS. 1 A-1B, according to some embodiments.
FIG. 4 is a flow diagram of a process for operating a therapy system and identifying if fluid contamination has occurred in the therapy system, according to some embodiments.
FIG. 5 is a graph showing oscillation frequency of the piezo-electric pump of FIGS. 1A-1B and 5-14 when the piezo-electric pump is not contaminated with fluid, according to some embodiments
FIG. 6 is a graph showing oscillation frequency of the piezo-electric pump of FIGS. 1A-1B and 5-14 when the piezo-electric pump is contaminated with fluid, according to some embodiments
FIG. 7 is a perspective view of the piezo-electric pump of FIGS. 1A-1B, according to some embodiments.
FIG. 8 is a perspective view of the piezo-electric pump of FIGS. 1A-1B, according to some embodiments.
FIG. 9 is a perspective view of the piezo-electric pump of FIGS. 1A-1B fluidly coupled with a bypass line, according to some embodiments.
FIG. 10 is a perspective view of the piezo-electric pump of FIGS. 1A-1B during a test, according to some embodiments.
FIG. 11 is a perspective view of the piezo-electric pump of FIGS. 1A-1B during the test, according to some embodiments. FIG. 12 is a perspective view of the piezo-electric pump of FIGS. 1A-1B when contaminated with fluid, according to some embodiments.
FIG. 13 is a perspective view of the piezo-electric pump of FIGS. 1A-1B and 5-10, according to some embodiments.
FIG. 14 is a top view of the piezo-electric pump of FIGS. 1A-1B and 5-10, according to some embodiments.

### DETAILED DESCRIPTION

### Overview

Referring generally to the FIGURES, a piezo-electric pump can be used to detect fluid contamination in a suction line or tubular member of a therapy system. The therapy system may include a therapy unit including a diaphragm pump that is configured to draw a negative pressure at a wound site through the suction line. The wound site may be covered with a dressing that defines an inner volume. The suction line fluidly couples the diaphragm pump with the inner volume. The therapy system also includes the piezo-electric pump for fluid detection that is fluidly coupled in parallel with the diaphragm pump (e.g., in a bypass line that fluidly couples with the suction line). The therapy unit also includes a controller that is configured to monitor feedback signals from the piezo-electric pump. The controller may be configured to determine if the piezo-electric pump has been contaminated with fluid based on the feedback signals. If the controller determines that the piezo-electric pump has been contaminated with fluid, the controller may cease operation of the diaphragm pump and/or provide an alert to a caregiver or a patient that fluid has entered or reached the piezo-electric pump. Advantageously, the systems and methods described herein can provide additional detectability to limit damage that may occur if fluid reaches various internal components of the therapy device.

### Therapy System

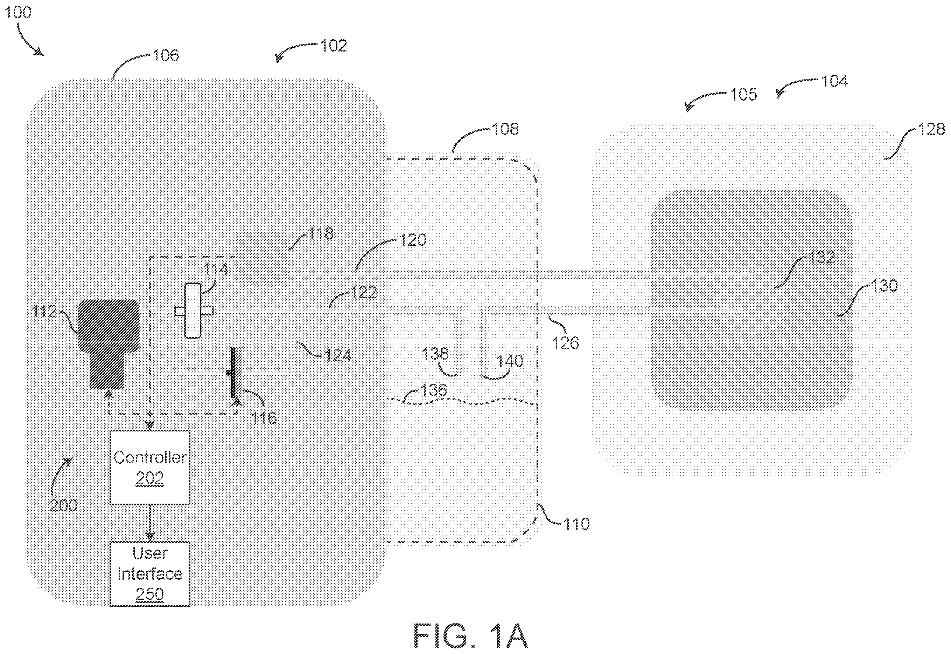

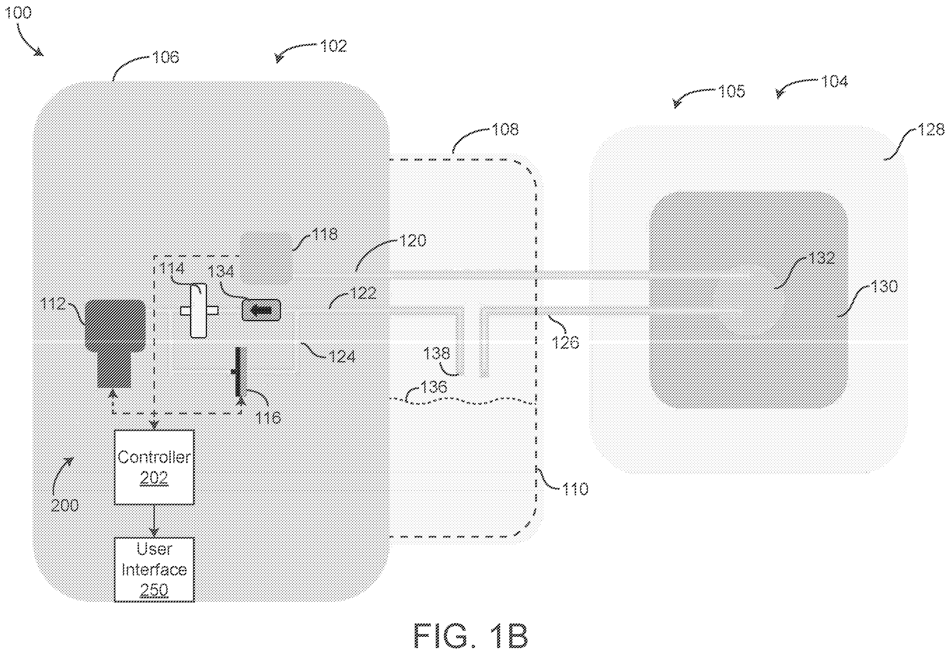

Referring now to FIGS. 1A and 1B, a therapy system 100 for providing negative pressure wound therapy (NPWT) to a wound site 105 is shown, according to some embodiments. Therapy system 100 includes a NPWT device 102 and a dressing 104 (e.g., a dressing assembly). NPWT device 102 is configured to draw a negative pressure at dressing 104 to facilitate healing of a wound at wound site 105, according to some embodiments. Dressing 104 can seal with peri-wound tissue or skin and define an inner volume. NPWT device 102 can operate to draw down or create a negative or reduced pressure within the inner volume of dressing 104, thereby providing negative pressure to the wound to facilitate healing progression of the wound.

Dressing 104 can include a wound drape 128, and an interface layer 130. Interface layer 130 can be a foam layer, an absorbent layer, etc., or any other material or structure that is configured to cover and/or engage the wound at wound site 105. In some embodiments, wound drape 128 is an outermost layer of dressing 104. Wound drape 128 can have an adhesive underside (e.g., an interior surface coated with an adhesive) for securing with peri-wound tissue. In some embodiments, wound drape 128 facilitates a fluidly sealed inner volume so that NPWT device 10 can draw a negative pressure within the inner volume. In some embodiments, NPWT device 102 is configured to control an operation of a V.A.C. VERAFLO^{™} Therapy, a PREVENA^{™} Therapy, an ABTHERA^{™} Open Abdomen Negative Pressure Therapy, or any other NPWT.

Referring still to FIGS. 1A-1B, therapy system 100 includes a first tubular member 120 (e.g., a conduit, a tube, a pipe, etc.), a second tubular member 122 (e.g., a conduit, a tube, a pipe, etc.), a third tubular member 126 (e.g., a conduit, a tube, a pipe, etc.), and a bypass tubular member 124 (e.g., a conduit, a tube, a pipe, etc.). NPWT device 102 includes a housing 106 and a canister 108. Canister 108 is fixedly coupled (e.g., removably) with the housing 106. The canister 108 includes an inner volume 110 that is configured to collect fluid (e.g., wound exudate) that is drawn from the wound site 105. The fluid may collect in the canister 108 until the fluid reaches a particular level (e.g., a full level). Once the canister 108 is filled with the fluid, the canister 108 may be emptied (e.g., removed from the housing 106 and emptied). The bypass tubular member 124 may be in a parallel configuration with the second tubular member 122.

The NPWT device 102 (e.g., the therapy unit) includes a pressure sensor 118, a filter 114, a diaphragm pump 112, a piezo-electric pump 116, and a control system 200. In some embodiments, the control system 200 includes a controller 202, the pressure sensor 118, the diaphragm pump 112, and the piezo-electric pump 116. The controller 202 is configured to obtain sensor information or feedback data from any of the pressure sensor 118 or the piezo-electric pump 116. The controller 202 is also configured to generate control signals for the diaphragm pump 112 (e.g., based on the sensor data/information or feedback data).

The diaphragm pump 112, the filter 114, the piezo-electric pump 116, and the pressure sensor 118 are positioned within the housing 106. The first tubular member 120 extends from an interfacing portion 132 of the dressing 104 to the pressure sensor 118. A first end of the first tubular member 120 is positioned within and fluidly couples with the inner volume of the dressing 104. A second end of the first tubular member 120 is positioned within the pressure sensor 118. In this way, a pressure within the first tubular member 120 is equal to a pressure within the inner volume of the dressing 104 so that the pressure sensor 118 can measure the pressure within the inner volume of the dressing 104.

The third tubular member 126 extends from the interfacing portion 132 of the dressing 104 to the inner volume 110 of the canister 108. The second tubular member 122 extends from the inner volume 110 of the canister 108 to the diaphragm pump 112. The third tubular member 126 includes an open end 140 that is positioned within the inner volume 110 of the canister 108. Similarly, the second tubular member 122 includes an open end 138 that is positioned within the inner volume 110 of the canister 108. The diaphragm pump 112 is configured to draw a negative pressure or a vacuum pressure through the second tubular member 122. The second tubular member 122 may terminate at the inner volume 110 of the canister 108 so that the inner volume 110 of the canister 108 is drawn down to the negative pressure or the vacuum pressure. The third tubular member 126 extends between the inner volume 110 of the canister 108 and the inner volume of the dressing 104 so that the inner volume of the dressing 104 is drawn down to the negative pressure or the vacuum pressure. In this way, the diaphragm pump 112, the second tubular member 122, and the third tubular member 126 can operate to draw a negative pressure or a vacuum pressure at the inner volume of the dressing 104. While the diaphragm pump 112 operates to draw the negative or vacuum pressure at the inner volume of the dressing 104, the pressure sensor 118 can monitor the negative or vacuum pressure within the inner volume of the dressing 104.

The piezo-electric pump 116 may also be operated simultaneously with operation of the diaphragm pump 112. The piezo-electric pump 116 may have less suction pressure than the diaphragm pump 112 and therefore does not necessarily significantly aid in drawing the negative pressure at the dressing 104. Rather, the piezo-electric pump 116 may operate to move air in the second tubular member 122 and the bypass tubular member 124. In this way, the piezo-electric pump 116 does not require pressure feedback to operate.

Due to the negative pressure drawn at the inner volume of the dressing 104 through the diaphragm pump 112, the second tubular member 122, and the third tubular member 126, fluid (e.g., wound exudate) can be driven to travel from the inner volume of the dressing 104, through the third tubular member 126, to the inner volume 110 of the canister 108. The fluid can then accumulate within the inner volume 110 of the canister 108. As the canister 108 fills with the fluid, a fluid surface 136 of the fluid may increase. Once the fluid surface 136 reaches the open end 138 of the second tubular member 122, the fluid may be transferred through the second tubular member 122. The second tubular member 122 includes the filter 114 positioned between the open end 138 of the second tubular member 122 and the diaphragm pump 112. The filter 114 is configured to filter air or fluid before it is provided to the diaphragm pump 112. The filter 114 can also function to prevent or limit fluid from reaching the diaphragm pump 112 if fluid enters the second tubular member 122. In some embodiments, the filter 114 is configured to increase an amount of time that fluid takes to reach the diaphragm pump 112 when fluid enters the second tubular member 122 (e.g., to delay fluid from reaching the diaphragm pump 112 and/or other electrical components of the NPWT device 102).

The bypass tubular member 124 extends between two positions on the second tubular member 122 and forms a bypass circuit. The bypass tubular member 124 fluidly couples with the second tubular member 122 at both positions. If the fluid surface 136 has not yet reached the open end 138 of the second tubular member 122, the diaphragm pump 112 may draw air through the second tubular member 122 (and therefore also the bypass tubular member 124) and the filter 114. As shown in FIG. 1B, the NPWT device 102 can also include a one-way valve 134 that is positioned upstream of the filter 114. The one-way valve 134 can be optional. The piezo-electric pump 116 is positioned along the bypass tubular member 124.

When the fluid surface 136 reaches the open end 138, the fluid may flow through the second tubular member 122 to the filter 114 and through the bypass tubular member 124. The piezo-electric pump 116 can be configured to provide feedback signals to the controller 202 which may indicate whether fluid has been introduced to the piezo-electric pump 116 through the bypass tubular member 124. For example, when only air is being passed through the piezo-electric pump 116, the feedback signals (e.g., voltage) may indicate that the piezo-electric pump 116 is not pumping water or a liquid. However, when liquid from the canister 108 is passed through the bypass tubular member 124, the feedback signals from the piezo-electric pump 116 may change, thereby indicating that fluid has entered the second tubular member 122 and the bypass tubular member 124. The controller 202 can monitor the feedback signals obtained from the piezo-electric pump 116 and determine, based on the feedback signals, if fluid has entered the second tubular member 122 and the bypass tubular member 124. The piezo-electric pump 116 can also function as a hydrophobic filter to limit or prevent fluids from reaching the diaphragm pump 112 and other high value or electrical components of the NPWT device 102.

If the fluid from the canister 108 is drawn through the second tubular member 122 and reaches the diaphragm pump 112, the fluid may damage the diaphragm pump 112, different pressure transducers, sensors, or circuitry of the NPWT device 102. In order to prevent damage from occurring to the NPWT device 102 or the various components thereof, the piezo-electric pump 116 can function as a sensor that samples the second tubular member 122 (e.g., a pressure line) to identify if fluid is present in the second tubular member 122 or not.

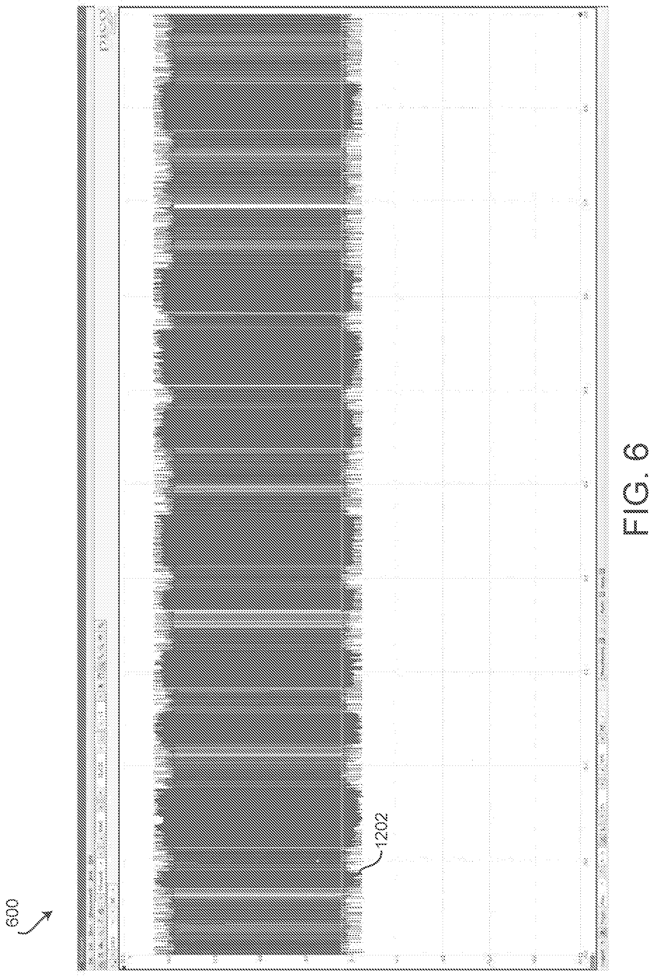

When fluid reaches the piezo-electric pump 116 (e.g., through the bypass tubular member 124), two possible outcomes may occur based on a volume and rate of introduction of the fluid. First, if the fluid is able to fully enter the piezo-electric pump 116, oscillation (e.g., voltage oscillation as indicated by the feedback signals) may cease completely. Second, if fluid reaches the piezo-electric pump 116 but does not stop the piezo-electric pump 116 completely, this can result in erratic oscillation frequency (e.g., as shown in FIG. 6). The controller 202 can be configured to identify either of these scenarios and thereby detect fluid contamination.

The piezo-electric pump 116 can also be used according to a negative pressure maintenance mode or as a negative pressure maintainer. For example, once the diaphragm pump 112 draws a negative pressure at the dressing 104, the piezo-electric pump 116 can operate to maintain the negative pressure at the dressing 104. This can reduce operation of the diaphragm pump 112 for maintaining the negative pressure at the dressing 104.

### Control System

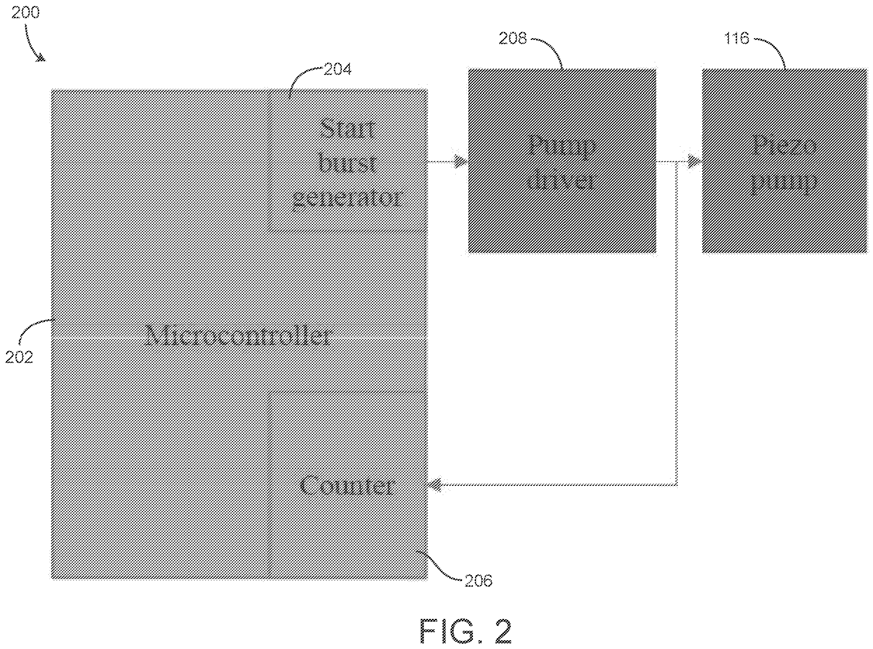

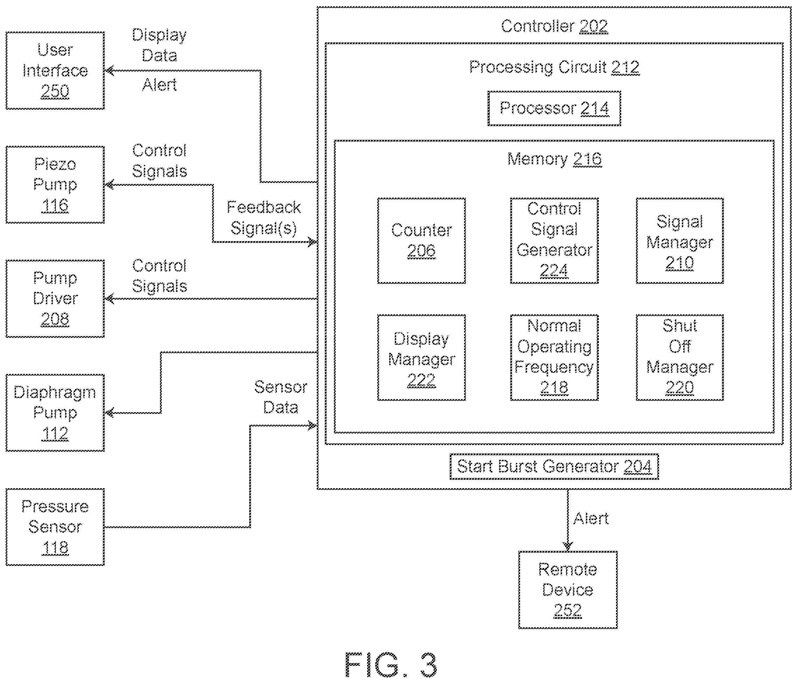

Referring now to FIGS. 2-3, the control system 200 is shown in greater detail, according to some embodiments. The control system 200 includes the controller 202 (e.g., a microcontroller), a pump driver 208, and the piezo-electric pump 116. The control system 200 also includes the diaphragm pump 112, and the pressure sensor 118. The control system 200 is configured to operate the pump driver 208 and/or the diaphragm pump 112 to draw a negative pressure at the wound site 105 based on the sensor data obtained from any of the pressure sensor 118, the piezo-electric pump 116, or any other sensing devices, units, sensors, etc. The control system 200 can be in communication with a remote device 252 (e.g., a remote system, a remote computing device, a caregiver system, a manufacturer system, etc.). In some embodiments, the remote device 252 is positioned remotely from the controller 202 or the control system 200 and is configured to receive alerts from the controller 202 that indicate if the piezo-electric pump 116 is not operating properly or if fluid has been detected by the piezo-electric pump 116.

The controller 202 is shown to include a processing circuit 212 including a processor 214 and memory 216. The processor 214 may be a general purpose single- or multi-chip processor, a digital signal processor (DSP), an application specific integrated circuit (ASIC), a field programmable gate array (FPGA), or other programmable logic device, discrete gate or transistor logic, discrete hardware components, or any combination thereof designed to perform the functions described herein. Processor 214 may be a microprocessor, or, any conventional processor, controller, microcontroller, or state machine. Processor 214 also may be implemented as a combination of computing devices, such as a combination of a DSP and a microprocessor, a plurality of microprocessors, one or more microprocessors in conjunction with a DSP core, or any other such configuration. In some embodiments, particular processes and methods may be performed by circuitry that is specific to a given function.

Memory 216 (e.g., memory, memory unit, storage device) may include one or more devices (e.g., RAM, ROM, Flash memory, hard disk storage) for storing data and/or computer code for completing or facilitating the various processes, layers and modules described in the present disclosure. Memory 216 may be or include volatile memory or non-volatile memory, and may include database components, object code components, script components, or any other type of information structure for supporting the various activities and information structures described herein. According to an exemplary embodiment, the memory 216 is communicably connected to the processor 214 via processing circuit 212 and includes computer code for executing (e.g., by the processing circuit or the processor) the one or more processes described herein.

Referring still to FIGS. 2-3, the memory 216 is shown to include a counter 206, a normal operating frequency 218, a signal manager 210, a display manager 222, a control signal generator 224, and a shut off manager 220. The counter 206 can be a frequency counter that is configured to monitor oscillation frequency of a circuit of the pump driver 208 and the piezo-electric pump 116. In some embodiments, the counter 206 is configured to monitor any issues with the piezo-electric pump 116 by identifying significant deviations in measured frequency with respect to normal operating frequency (e.g., the normal operating frequency 218 stored in the memory 216) and/or by identifying failure of the piezo-electric pump 116 to oscillate. In some embodiments, the oscillating frequency of the piezo-electric pump 116 is determined by counter 206 and/or signal manager 210 based on resonant frequency of the piezo-electric pump 116 (e.g., approximately 23-24 kHz). The resonant frequency of the piezo-electric pump 116 and therefore the operating frequency of the piezo-electric pump 116 may vary or be dependent on load and temperature of the piezo-electric pump 116.

Referring still to FIGS. 2-3, the controller 202 is shown to include a start burst generator 204. The start burst generator 204 is configured to generate a 24 kHz input signal that is applied to the piezo-electric pump 116 to facilitate rapid and reliable starting of the piezo-electric pump 116.

Referring still to FIGS. 2-3, the counter 206 is configured to count real-time, current, actual, or measured oscillation frequency of the piezo-electric pump 116. The counter 206 can count an actual oscillation frequency *f_{actual}* based on the feedback signal(s) obtained from the piezo-electric pump 116. The counter 206 may provide the actual oscillation frequency *f_{actual}* to the signal manager 210 for analysis of the actual oscillation frequency *f_{actual}.*

Referring still to FIGS. 2-3, the signal manager 210 is configured to obtain the actual oscillation frequency *f_{actual}* from the counter 206, and a normal operating oscillation frequency *f_{operating}* from the normal operating frequency 218. The normal operating frequency 218 may be a stored parameter of the normal oscillation frequency *f_{operating}* (e.g., given certain conditions such as load and temperature, which may also be detected and provided to the controller 202 to augment or adjust the actual oscillation frequency *f_{actual}*) or a stored graph (e.g., as shown in graph 600 in FIG. 6) of normal or expected operational oscillations of the piezo-electric pump 116.

The signal manager 210 can compare the actual oscillation frequency *f_{actual}* to the operating oscillation frequency *f_{operating}* to determine if the piezo-electric pump 116 is operating normally (and thereby to determine if the piezo-electric pump 116 is free of fluid) or to determine if the piezo-electric pump 116 is not operating normally (due to the piezo-electric pump 116 being contaminated with fluid or due to the piezo-electric pump 116 failing). For example, the signal manager 210 may compare the actual oscillation frequency *f_{actual}* to the operating oscillation frequency *f_{operating}* to determine if the actual oscillation frequency *f_{actual}* is greater than or less than the operating oscillation frequency *f_{operating}.* If the actual oscillation frequency *f_{actual}* is greater than the operating oscillation frequency *f_{operating}* (e.g., *f_{actual}* > *f_{operating}*), the signal manager 210 can determine that the piezo-electric pump 116 has been contaminated with fluid. In some embodiments, the signal manager 210 determines if the actual oscillation frequency *f_{actual}* is greater than (or less than) the operating oscillation frequency *f_{operating}* by a predetermined amount, by a percentage amount, by a standard deviation, by a portion of a standard deviation, by multiple standard deviations, etc. If the actual oscillation frequency *f_{actual}* is greater than (or less than) the actual oscillation frequency *f_{actual}* by a significant amount, the signal manager 210 may determine that the piezo-electric pump 116 is contaminated with fluid, and can provide an indication to the shut off manager 220, the display manager 222, and/or the control signal generator 224.

If the signal manager 210 determines that the actual oscillation frequency *f_{actual}* is substantially equal to or the same as the operating oscillation frequency *f_{operating},* the signal manager 210 may determine that the piezo-electric pump 116 has not been contaminated with fluid and is operating properly. The signal manager 210 may provide a notification indicating that the piezo-electric pump 116 has not been contaminated with fluid or is operating properly to any of the control signal generator 224, the display manager 222, or the shut off manager 220.

In some embodiments, the signal manager 210 can also determine if the piezo-electric pump 116 is approaching failure based on the feedback signal(s), or more particularly based on the actual oscillation frequency *f_{actual}.* For example, the signal manager 210 can determine if the actual oscillation frequency *f_{actual}* is approaching zero, is significantly less than the actual oscillation frequency *f_{actual}* (e.g., less than the operating oscillation frequency *f_{operating}* by a percentage, a predetermined amount, a standard deviation, a portion of a standard deviation, multiple standard deviations, etc.), and thereby determine that the piezo-electric pump 116 has failed or is predicted to fail soon.

The shut off manager 220 can receive outputs of the signal manager 210 (e.g., an indication of whether the piezo-electric pump 116 is operating normally, an indication of whether the piezo-electric pump 116 has been contaminated with fluid, or an indication of whether the piezo-electric pump 116 has failed or is predicted to fail soon). If the outputs from the signal manager 210 indicate that the piezo-electric pump 116 has failed, is predicted to fail, or has been contaminated with fluid, the shut off manager 220 may generate a shut-off signal and provide the shut-off signal to the control signal generator 224, or directly to the diaphragm pump 112. The shut-off signal is provided to the diaphragm pump 112 to shut off the diaphragm pump 112 to thereby prevent damage to the piezo-electric pump 116 (e.g., if the piezo-electric pump 116 is predicted to fail soon) or to prevent leakage from occurring (e.g., if the piezo-electric pump 116 has been contaminated with fluid).

Advantageously, the piezo-electric pump 116 can function both as a pump (e.g., to draw a negative pressure at the wound site 105) and as a fluid indicator or sensor (e.g., to notify the controller 202 when fluid is present at the piezo-electric pump 116 based on the feedback signal(s)). Based on fluid indication as determined based on the feedback signal(s) obtained from the piezo-electric pump 116, the controller 202 may shut off the diaphragm pump 112 and notify a user or a caregiver (e.g., via the display manager 222) that the diaphragm pump 112 has been shut off, and a reason why the diaphragm pump 112 has been shut off (e.g., due to fluid contamination at the piezo-electric pump 116, due to the piezo-electric pump 116 failing or being predicted to fail soon, etc.). In some embodiments, analysis of the feedback signal(s) allows the controller 202 to shut off the diaphragm pump 112 once the piezo-electric pump 116 has been contaminated with fluid but before the diaphragm pump 112 or various electrical components of the NPWT device 102 have been contaminated with fluid.

Referring still to FIGS. 2-3, the memory 216 includes the display manager 222 that is configured to generate display data for a user interface 250 and operate the user interface 250 (e.g., a display screen) to provide the display data to the caregiver. The display data can include an indication of whether the piezo-electric pump 116 is currently operating (e.g., whether or not a shut off signal has been provided to the piezo-electric pump 116), whether the piezo-electric pump 116 is contaminated with fluid or not, operational parameters of the piezo-electric pump 116 and/or the diaphragm pump 112, a current pressure within the dressing 104 (e.g., pressure as detected by the pressure sensor 118), graphs displaying a drawdown or target pressure, the operating oscillation frequency *f_{operating}* of the piezo-electric pump 116, the actual oscillation frequency *f_{actual}* of the piezo-electric pump 116, etc. The display manager 222 may communicate with any of the user interface 250, the piezo-electric pump 116, the pump driver 208, the diaphragm pump 112, the pressure sensor 118, the counter 206, the control signal generator 224, the signal manager 210, the normal operating frequency 218, or the shut-off manager 220 to collect data and generate the display data for display on the user interface 250.

In some embodiments, the display manager 222 is also configured to facilitate communication with the remote device 252. For example, the remote device 252 may be a caregiver system, a hospital system, a manufacturer system, or device thereof that is configured to communicate with the controller 202. The remote device 252 can notify the remote device 252 (e.g., and thereby a caregiver, a medical or health professional, a hospital system, etc.) when the piezo-electric pump 116 operates abnormally (e.g., when actual oscillation frequency *f_{actua}* exceeds the operating oscillation frequency *f_{operating}*) or when the piezo-electric pump 116 detects fluid contamination. The controller 202 and the remote device 252 can be configured to communicate wirelessly (e.g., using a cellular network, the Internet, etc.). The display manager 222 can also provide any of the display data that is provided to the user interface 250 to the remote device 252 for remote access or viewing.

Referring still to FIGS. 2-3, the controller 202 includes the control signal generator 224, according to some embodiments. The control signal generator 224 is configured to obtain or receive sensor data (e.g., pressure) from the pressure sensor 118. The pressure obtained from the pressure sensor 118 is a reading of pressure within the inner volume of the dressing 104. The control signal generator 224 can implement a control scheme (e.g., a closed loop control scheme, a PID control scheme, etc.) to generate control signals for the diaphragm pump 112 to draw the negative pressure at the inner volume of the dressing 104. The control signal generator 224 may also generate control signals for the piezo-electric pump 116 to aid in drawing negative pressure at the inner volume of the dressing 104. In some embodiments, the control signal generator 224 operates both the diaphragm pump 112 and the piezo-electric pump 116 concurrently or simultaneously to draw the negative pressure at the inner volume of the dressing 104 (e.g., depending on a mode of the NPWT device 102). In some embodiments, the control signal generator 224 is configured to operate the diaphragm pump 112 and the piezo-electric pump 116 independently from each other (e.g., based on a mode of the NPWT device 102).

For example, the NPWT device 102 may operate according to a first mode, a second mode, and a third mode. In the first mode, the control signal generator 224 may generate control signals only for the diaphragm pump 112 so the diaphragm pump 112 operates to draw the negative pressure at the inner volume of the dressing 104. In the second mode, the control signal generator 224 may generate control signals for both the diaphragm pump 112 and the piezo-electric pump 116 so that both the diaphragm pump 112 and the piezo-electric pump 116 operate to draw the negative pressure at the dressing 104. In the third mode (e.g., a silent suction mode), the control signal generator 224 may generate control signals only for the piezo-electric pump 116 so that the piezo-electric pump 116 operates to draw the negative pressure at the dressing 104, without operating the diaphragm pump 112. In some embodiments, an additional piezo-electric pump is configured (e.g., similarly to the diaphragm pump 112) to draw a negative pressure at the dressing 104 and the controller 202 operates the additional piezo-electric pump to draw the negative pressure at the dressing 104. The mode may be selected by a user or a caregiver via the user interface 250 and provided to the controller 202 so that the control signal generator 224 operates the piezo-electric pump 116, the diaphragm pump 112, and/or the additional piezo-electric pump according to the selected mode.

### Process

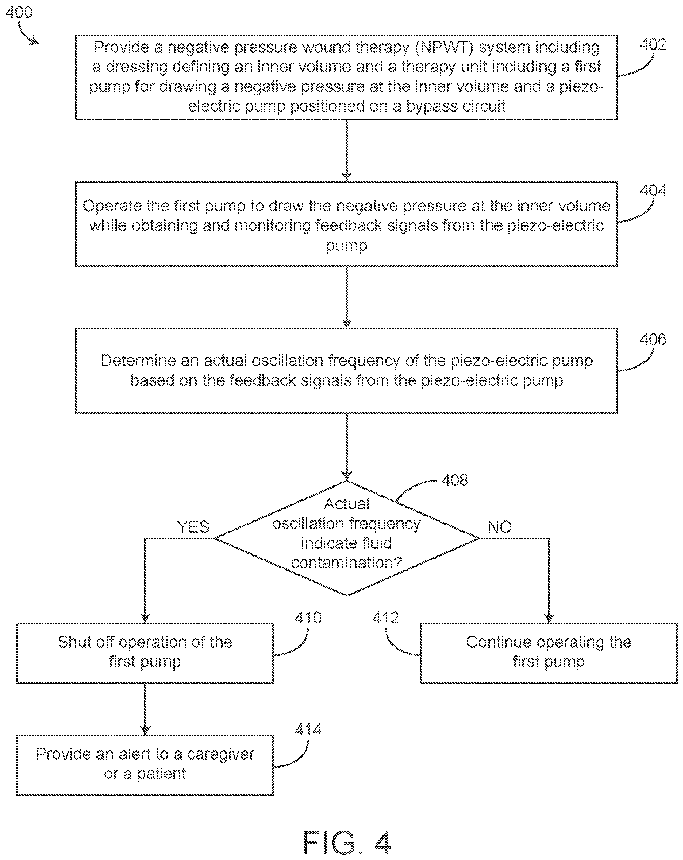

Referring now to FIG. 4, a flow diagram of a process 400 for controlling operation of a therapy system or a NPWT device including a dual pump arrangement is shown, according to some embodiments. The process 400 includes steps 402-414 and can be performed by the therapy system 100 or the various components thereof. Process 400 facilitates preventing or limiting a likelihood of a leak occurring at the NPWT device.

Process 400 includes providing a NPWT system including a dressing defining an inner volume and a therapy unit including a first pump for drawing a negative pressure at the inner volume and a piezo-electric pump positioned on a bypass circuit (step 402), according to some embodiments. The NPWT system may be the therapy system 100 as described in greater detail above. The first pump may be the diaphragm pump 112 or any other pump that is configured to provide primary negative pressure draw down at a wound dressing that is configured to seal with periwound skin and define the inner volume. The piezo-electric pump may be the piezo-electric pump 116 that is positioned along the bypass tubular member 124. The piezo-electric pump can be configured to identify if fluid has entered the therapy unit (e.g., when the canister becomes full or when the first pump draws fluid into the therapy unit). The NPWT system can also include a controller that is configured to obtain sensor data and/or feedback data (e.g., from sensors or from the piezo-electric pump). The controller may also be configured to perform a control scheme to operate the first pump to draw the negative pressure at the inner volume.

Process 400 includes operating the first pump to draw the negative pressure at the inner volume while obtaining and monitoring feedback signals from the piezo-electric pump (step 404), according to some embodiments. Step 404 can be performed by the controller of the NPWT system, or more particularly by the controller 202. In some embodiments, step 404 is performed by the control signal generator 224, the counter 206, and the signal manager 210.

Process 400 includes determining an actual oscillation frequency of the piezo-electric pump based on the feedback signals obtained from the piezo-electric pump (step 406), according to some embodiments. Step 406 can be performed by the counter 206 based on the feedback signal(s) obtained from the piezo-electric pump 116. For example, the counter 206 may count oscillations of the feedback signal(s) obtained from the piezo-electric pump 116 over a time interval to determine the actual oscillation frequency of the piezo-electric pump 116.

Process 400 includes determining if the actual oscillation frequency indicates fluid contamination (step 408), according to some embodiments. In some embodiments, step 408 includes comparing the actual oscillation frequency (e.g., *f_{actual}*) to an operating oscillation frequency (e.g., *f_{operating}*) to determine if the piezo-electric pump is operating normally and/or to determine if the piezo-electric pump has been contaminated with fluid (thereby indicating that fluid has reached the therapy device). In some embodiments, step 408 is performed by the signal manager 210 based on the actual oscillation frequency (e.g., as determined by the counter 206) and a value of the operating oscillation frequency that is stored in the memory 216 of the controller 202. For example, the signal manager 210 may compare the actual oscillation frequency to the operating oscillation frequency, and if the actual oscillation frequency exceeds the operating oscillation frequency by a significant amount (e.g., by a predetermined amount, by a statistically significant amount, etc.) then the signal manager 210 may determine that the actual oscillation frequency indicates that the piezo-electric pump has been contaminated with the fluid (step 408, "YES") and may proceed to step 410. If the actual oscillation frequency is substantially equal to the operating oscillation frequency (step 408, "NO"), process 400 may proceed to step 412.

Process 400 includes shutting off the first pump (step 410) in response to the actual oscillation frequency indicating fluid contamination of the piezo-electric pump and/or indicating that fluid has reached the therapy unit, according to some embodiments. Step 410 can be performed in response to step 408 if the actual oscillation frequency indicates fluid contamination (step 408, "YES"). In some embodiments, step 410 is performed by the shut off manager 220 and/or the control signal generator 224. Step 410 can include ceasing to provide control signals to the first pump and/or providing a shut-off signal to the first pump to shut off operation of the first pump.

Process 400 includes continuing operation of the first pump (step 412) in response to the actual oscillation frequency not indicating fluid contamination (step 408, "NO"), according to some embodiments. In some embodiments, step 412 includes returning to step 404 in response to determining that the actual oscillation frequency does not indicate fluid contamination (step 408, "NO"). Step 412 can include continuing to monitor feedback signals and/or oscillation frequency obtained from the piezo-electric pump as the first pump is operated to draw the negative pressure.

Process 400 includes providing an alert to a caregiver or a patient (step 414), according to some embodiments. Step 414 can be performed by the controller 202, or more particularly, by the display manager 222. For example, the alert may be provided in the form of display data, a message, a notification, a visual alert, an aural alert, etc., via the user interface 250 (e.g., a local alert) and/or the remote device 252 (e.g., a remote alert). In this way, detection of fluid contamination can be reported to a user or patient (e.g., via the user interface 250) and/or reported to a caregiver or a hospital system (e.g., via the remote device 252).

### Oscillation Graphs

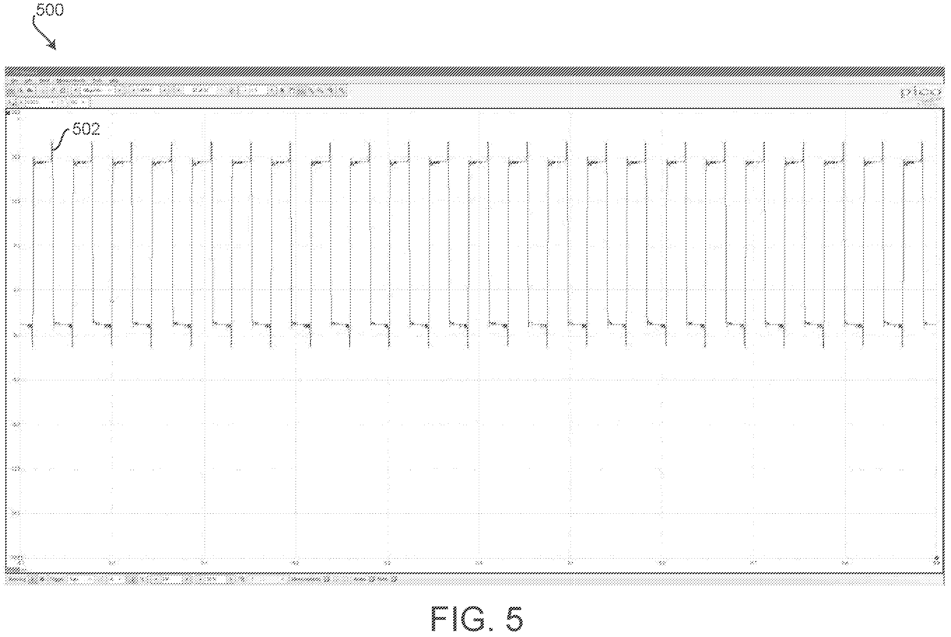

Referring now to FIGS. 5-6, graphs 500 and 600 illustrate feedback signal(s) obtained from the piezo-electric pump 116. Graphs 500 and 600 can be generated by the controller 202 and may be provided or displayed on the user interface 250. Graph 500 includes series 502 that illustrates oscillations of voltage across the piezo-electric pump 116 over time, when the piezo-electric pump 116 is operating normally (e.g., when the piezo-electric pump 116 is not contaminated with fluid). Graph 600 includes series 602 that illustrates oscillations of voltage across the piezo-electric pump 116 over time, when the piezo-electric pump 116 is not operating normally (e.g., when the piezo-electric pump 116 is contaminated with fluid). As shown by comparing the graph 500 and the graph 600, the piezo-electric pump 116 experiences increased oscillations (e.g., increased oscillation frequency) when the piezo-electric pump 116 is contaminated with fluid. This enables the controller 202 to analyze the feedback signals obtained from the piezo-electric pump 116 to determine if the piezo-electric pump 116 has been contaminated with fluid. The controller 202 can determine that the piezo-electric pump 116 has been contaminated using statistical analysis (e.g., to identify noise or variance in the feedback signals), an oscillation frequency of the feedback signals of the piezo-electric pump 116, or by comparing the feedback signals of the piezo-electric pump 116 to a known template or known signal dataset (e.g., where deviations from the known template or known signal dataset indicate that the piezo-electric pump 116 is not operating properly such as due to fluid contamination). In this way, the piezo-electric pump 116 can function as a fluid indicator, and the feedback signals may indicate when fluid is present in the bypass tubular member 124. As shown in FIG. 6, pump oscillation frequency alternate between relatively normal operation (at approximately 24kHz) and higher frequencies (in the order of 30 kHz). As shown in FIG. 6, short periods of non-oscillation may also be observed.

### Piezo-Electric Pump

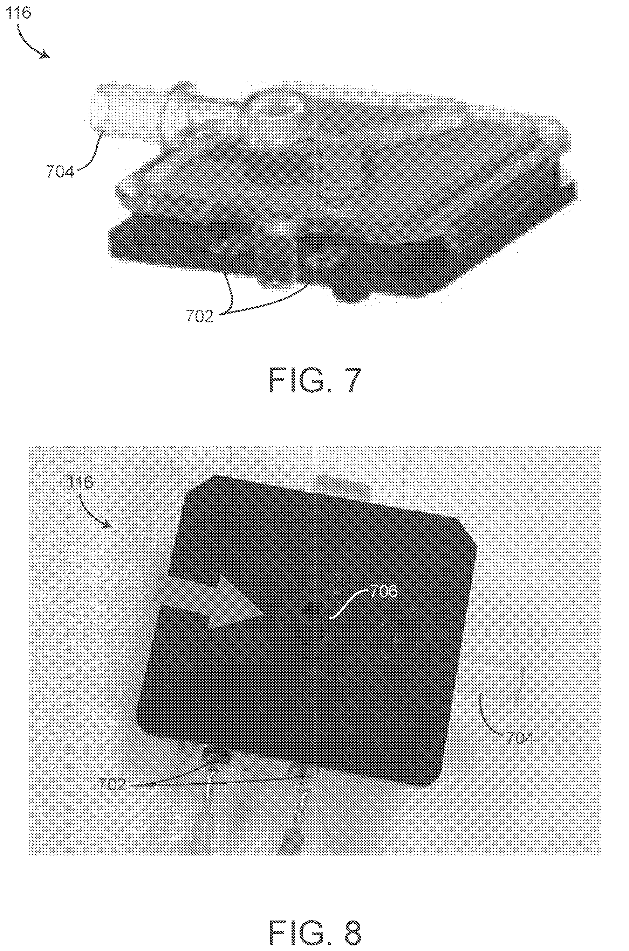

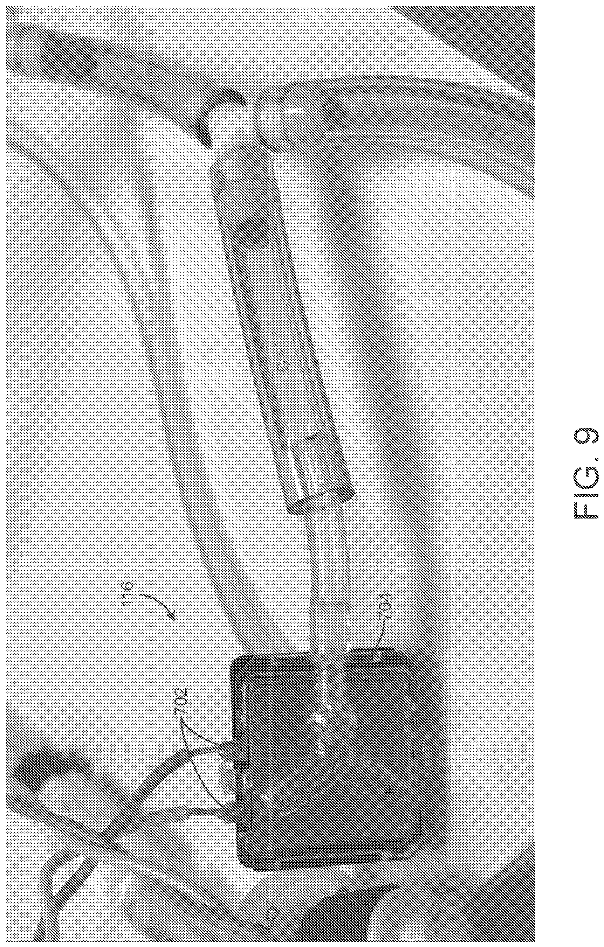

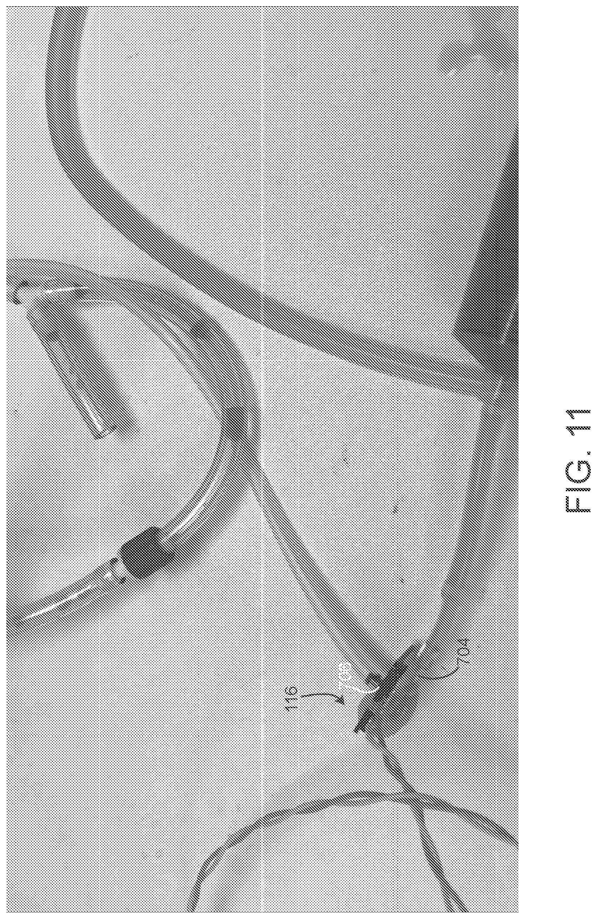

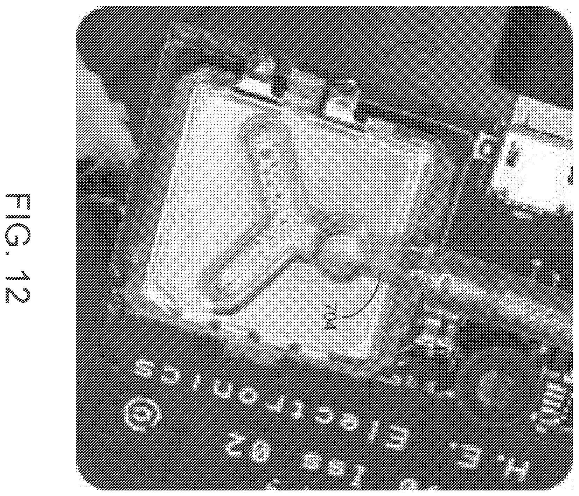

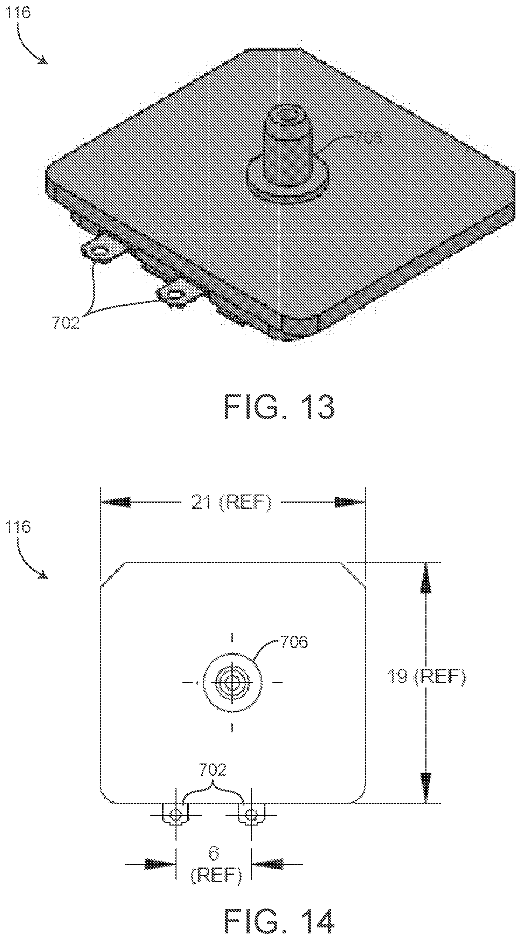

Referring now to FIGS. 7-14, the piezo-electric pump 116 is shown in greater detail, according to an exemplary embodiment. The piezo-electric pump 116 can operate at a resonant device driven by a +/- 15-18 volt square wave that is supplied by a driver that forms a portion of an oscillation circuit. As shown in FIGS. 7-14, the piezo-electric pump 116 may include a rear hole 706 that functions as a back-up intake line for the piezo-electric pump 116. In some embodiments, the rear hole 706 is plugged when the piezo-electric pump 116 is implemented in the NPWT device 102. The piezo-electric pump 116 can also include an opening 704 where the bypass tubular member 124 is fluidly coupled so that the piezo-electric pump 116 is configured to draw negative pressure through the bypass tubular member 124.

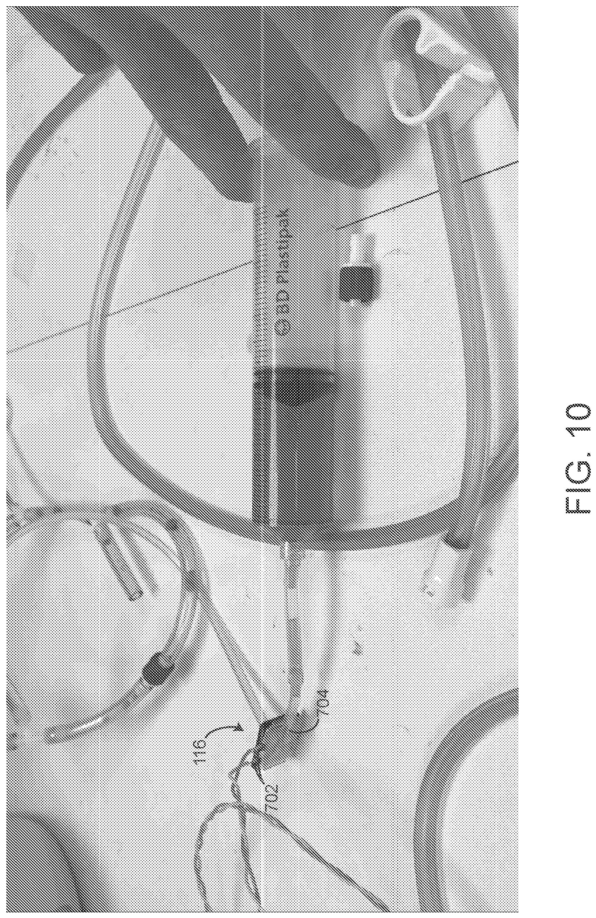

FIGS. 10-11 show test results from injecting fluid into the piezo-electric pump 116 through the opening 704. From the tests shown in FIGS. 10-11, it was determined that the piezo-electric pump 116 would not experience a pressure during normal operation of the therapy system 100. Therefore, the piezo-electric pump 116 can be used in the therapy system 100 to detect fluid contamination without exhausting liquid that could damage components of the NPWT device 102.

### Advantages

Specifically, the systems and methods described herein can be used to limit or prevent fluid ingress to a capital device (e.g., the NPWT device 102). The piezo-electric pump 116 can be used to detect if fluid enters the bypass tubular member 124, and the NPWT device 102 may alert a caregiver so that the caregiver can repair or replace the NPWT device 102. Additionally, the NPWT device 102 may shut-off therapy when the piezo-electric pump 116 detects fluid contamination. The systems and methods described herein may reduce repair cost, and reduce a number of components that may require replacement. Additionally, the systems and methods described herein can reduce repair time, and remove a risk of electrical shortage within the NPWT device 102 due to fluid ingress to internal circuitry of the NPWT device 102.

### Configuration of Exemplary Embodiments

As utilized herein, the terms "approximately," "about," "substantially", and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and claimed are considered to be within the scope of the disclosure as recited in the appended claims.

It should be noted that the term "exemplary" and variations thereof, as used herein to describe various embodiments, are intended to indicate that such embodiments are possible examples, representations, or illustrations of possible embodiments (and such terms are not intended to connote that such embodiments are necessarily extraordinary or superlative examples).

The hardware and data processing components used to implement the various processes, operations, illustrative logics, logical blocks, modules and circuits described in connection with the embodiments disclosed herein may be implemented or performed with a general purpose single- or multi-chip processor, a digital signal processor (DSP), an application specific integrated circuit (ASIC), a field programmable gate array (FPGA), or other programmable logic device, discrete gate or transistor logic, discrete hardware components, or any combination thereof designed to perform the functions described herein. A general purpose processor may be a microprocessor, or, any conventional processor, controller, microcontroller, or state machine. A processor also may be implemented as a combination of computing devices, such as a combination of a DSP and a microprocessor, a plurality of microprocessors, one or more microprocessors in conjunction with a DSP core, or any other such configuration. In some embodiments, particular processes and methods may be performed by circuitry that is specific to a given function. The memory (e.g., memory, memory unit, storage device) may include one or more devices (e.g., RAM, ROM, Flash memory, hard disk storage) for storing data and/or computer code for completing or facilitating the various processes, layers and modules described in the present disclosure. The memory may be or include volatile memory or non-volatile memory, and may include database components, object code components, script components, or any other type of information structure for supporting the various activities and information structures described in the present disclosure. According to an exemplary embodiment, the memory is communicably connected to the processor via a processing circuit and includes computer code for executing (e.g., by the processing circuit or the processor) the one or more processes described herein.

The present disclosure contemplates methods, systems and program products on any machine-readable media for accomplishing various operations. The embodiments of the present disclosure may be implemented using existing computer processors, or by a special purpose computer processor for an appropriate system, incorporated for this or another purpose, or by a hardwired system. Embodiments within the scope of the present disclosure include program products comprising machine-readable media for carrying or having machine-executable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, or other optical disk storage, magnetic disk storage or other magnetic storage devices, or any other medium which can be used to carry or store desired program code in the form of machine-executable instructions or data structures and which can be accessed by a general purpose or special purpose computer or other machine with a processor. Combinations of the above are also included within the scope of machine-readable media. Machine-executable instructions include, for example, instructions and data which cause a general purpose computer, special purpose computer, or special purpose processing machines to perform a certain function or group of functions.

## Claims

1. A therapy unit for a negative pressure wound therapy, NPWT, system, the therapy unit comprising:
a first pump (112) configured to draw a negative pressure at a wound site through a first tubular member (122);
a piezo-electric pump (116) fluidly coupled with the first tubular member (122) through a bypass tubular member (124) and configured to detect a fluid indication; and
a controller (202) comprising processing circuitry configured to:
obtain feedback signals from the piezo-electric pump (116); and
determine if fluid is present at the piezo-electric pump (116) based on the feedback signals.

2. The therapy unit of Claim 1, wherein the feedback signals obtained from the piezo-electric pump (116) comprise a voltage across the piezo-electric pump (116).

3. The therapy unit Claim 1, wherein the processing circuitry of the controller (202) is further configured to shut off operation of the first pump (112) in response to determining that fluid is present at the piezo-electric pump (116).

4. The therapy unit of Claim 1, wherein the processing circuitry of the controller (202) is configured to notify a caregiver in response to determining that fluid is present at the piezo-electric pump (116).

5. The therapy unit of Claim 1, wherein the processing circuitry of the controller (202) is configured to:
determine an oscillation frequency of the piezo-electric pump (116) based on the feedback signals of the piezo-electric pump (116); and
determine if fluid is present at the piezo-electric pump (116) based on the oscillation frequency.

6. The therapy unit of Claim 5, wherein the processing circuitry of the controller (202) is configured to compare the oscillation frequency of the piezo-electric pump (116) to a predetermined oscillation frequency value and determine that fluid is present at the piezo-electric pump (116) in response to the oscillation frequency exceeding the predetermined oscillation frequency value.

7. The therapy unit of Claim 5, wherein the processing circuitry of the controller (202) is configured to determine if the piezo-electric pump (116) is inoperational based on the oscillation frequency.

8. The therapy unit of Claim 5, further comprising a pressure sensor (118) fluidly coupled with the wound site and configured to read a pressure at the wound site, wherein the processing circuitry of the controller (202) is configured to operate the first pump (112) to draw the negative pressure at the wound site based on the pressure at the wound site.

9. The therapy unit of Claim 8, wherein the controller is (202) configured to operate the first pump (112) and the piezo-electric pump (116) simultaneously.

10. The therapy unit of Claim 8, wherein the first pump (112) is a diaphragm pump.

11. The therapy unit of Claim 1 , further comprising a housing (106), wherein the diaphragm pump (112) and the piezo-electric pump (116) are positioned within the housing (106).

12. The therapy unit of Claim 1, further comprising a canister (108) configured to collect fluid drawn from the wound site due to operation of the first pump (112).

13. A negative pressure wound therapy system comprising a therapy unit according to any of claims 1 to 12.

## Patentansprüche

1. Eine Therapieeinheit für ein Unterdruckwundtherapiesystem, NPWT, die Therapieeinheit aufweisend:
eine erste Pumpe (112), die konfiguriert ist, um einen Unterdruck an einer Wundstelle durch ein erstes schlauchförmiges Element (122) zu erzeugen;
eine piezoelektrische Pumpe (116), die durch ein schlauchförmiges Bypass-Element (124) mit dem ersten schlauchförmigen Element (122) fluidisch gekoppelt ist und konfiguriert ist, um eine Fluidanzeige zu erkennen; und
eine Steuerung (202), aufweisend einen Verarbeitungsschaltkreis, der konfiguriert ist zum:
Erhalten von Rückkopplungssignalen von der piezoelektrischen Pumpe (116); und
Bestimmen, ob Fluid an der piezoelektrischen Pumpe (116) vorhanden ist, basierend auf den Rückkopplungssignalen.

2. Die Therapieeinheit nach Anspruch 1, wobei die Rückkopplungssignale, die von der piezoelektrischen Pumpe (116) erhalten werden, eine Spannung über der piezoelektrischen Pumpe (116) aufweisen.

3. Die Therapieeinheit nach Anspruch 1, wobei der Verarbeitungsschaltkreis der Steuerung (202) ferner konfiguriert ist, um einen Betrieb der ersten Pumpe (112) als Reaktion auf das Bestimmen, dass Fluid an der piezoelektrischen Pumpe (116) vorhanden ist, abzuschalten.

4. Die Therapieeinheit nach Anspruch 1, wobei der Verarbeitungsschaltkreis der Steuerung (202) konfiguriert ist, um eine Pflegekraft zu benachrichtigen als Reaktion auf das Bestimmen, dass Fluid an der piezoelektrischen Pumpe (116) vorhanden ist.

5. Die Therapieeinheit nach Anspruch 1, wobei der Verarbeitungsschaltkreis der Steuerung (202) konfiguriert ist zum:
Bestimmen einer Schwingungsfrequenz der piezoelektrischen Pumpe (116) basierend auf den Rückkopplungssignalen der piezoelektrischen Pumpe (116); und
Bestimmen, ob Fluid an der piezoelektrischen Pumpe (116) vorhanden ist, basierend auf der Schwingungsfrequenz.

6. Die Therapieeinheit nach Anspruch 5, wobei der Verarbeitungsschaltkreis der Steuerung (202) konfiguriert ist, um die Schwingungsfrequenz der piezoelektrischen Pumpe (116) mit einem vorbestimmten Schwingungsfrequenzwert zu vergleichen und zu bestimmen, dass Fluid an der piezoelektrischen Pumpe (116) vorhanden ist, als Reaktion darauf, dass die Schwingungsfrequenz den vorbestimmten Schwingungsfrequenzwert überschreitet.

7. Die Therapieeinheit nach Anspruch 5, wobei der Verarbeitungsschaltkreis der Steuerung (202) konfiguriert ist, um zu bestimmen, ob die piezoelektrische Pumpe (116) nicht betriebsfähig ist, basierend auf der Schwingungsfrequenz.

8. Die Therapieeinheit nach Anspruch 5, ferner aufweisend einen Drucksensor (118), der mit der Wundstelle fluidisch gekoppelt ist und konfiguriert ist, um einen Druck an der Wundstelle zu lesen, wobei der Verarbeitungsschaltkreis der Steuerung (202) konfiguriert ist, um die erste Pumpe (112) zu betreiben, um den Unterdruck an der Wundstelle basierend auf dem Druck an der Wundstelle zu erzeugen.

9. Die Therapieeinheit nach Anspruch 8, wobei die Steuerung (202) konfiguriert ist, um die erste Pumpe (112) und die piezoelektrische Pumpe (116) gleichzeitig zu betreiben.

10. Die Therapieeinheit nach Anspruch 8, wobei die erste Pumpe (112) eine Membranpumpe ist.

11. Die Therapieeinheit nach Anspruch 1, ferner aufweisend ein Gehäuse (106), wobei die Membranpumpe (112) und die piezoelektrische Pumpe (116) innerhalb des Gehäuses (106) positioniert sind.

12. Die Therapieeinheit nach Anspruch 1, ferner aufweisend einen Behälter (108), der konfiguriert ist, um Fluid zu sammeln, das aufgrund des Betriebs der ersten Pumpe (112) aus der Wundstelle erzeugt wird.

13. Ein Unterdruckwundtherapiesystem, aufweisend eine Therapieeinheit nach einem der Ansprüche 1 bis 12.

## Revendications

1. Unité de thérapie pour un système de thérapie de plaie par pression négative, NPWT, l'unité de thérapie comprenant :
une première pompe (112) conçue pour aspirer une pression négative au niveau d'un site de plaie à travers un premier élément tubulaire (122) ;
une pompe piézoélectrique (116) accouplée fluidiquement au premier élément tubulaire (122) par l'intermédiaire d'un élément tubulaire de dérivation (124) et configurée pour détecter une indication de fluide ; et
un dispositif de commande (202) comprenant un ensemble de circuits de traitement configuré pour :
obtenir des signaux de rétroaction de la pompe piézoélectrique (116) ; et
déterminer la présence de fluide au niveau de la pompe piézo-électrique (116) sur la base des signaux de rétroaction.

2. Unité de thérapie selon la revendication 1, dans laquelle les signaux de rétroaction obtenus à partir de la pompe piézoélectrique (116) comprennent une tension aux bornes de la pompe piézoélectrique (116).

3. Unité de thérapie selon la revendication 1, dans laquelle l'ensemble de circuits de traitement du dispositif de commande (202) est en outre configuré pour interrompre le fonctionnement de la première pompe (112) en réponse à la détermination de la présence de fluide au niveau de la pompe piézoélectrique (116).

4. Unité de thérapie selon la revendication 1, dans laquelle l'ensemble de circuits de traitement du dispositif de commande (202) est configuré pour avertir un soignant en réponse à la détermination de la présence d'un fluide au niveau de la pompe piézo-électrique (116).

5. Unité de thérapie selon la revendication 1, dans laquelle l'ensemble de circuits de traitement du dispositif de commande (202) est configuré pour :
déterminer une fréquence d'oscillation de la pompe piézoélectrique (116) sur la base des signaux de rétroaction de la pompe piézoélectrique (116) ; et
déterminer la présence de fluide au niveau de la pompe piézoélectrique (116) sur la base de la fréquence d'oscillation.

6. Unité de thérapie selon la revendication 5, dans laquelle l'ensemble de circuits de traitement du dispositif de commande (202) sont configurés pour comparer la fréquence d'oscillation de la pompe piézoélectrique (116) à une valeur de fréquence d'oscillation prédéterminée et déterminer qu'un fluide est présent au niveau de la pompe piézoélectrique (116) en réponse à la fréquence d'oscillation dépassant la valeur de fréquence d'oscillation prédéterminée.

7. Unité de thérapie selon la revendication 5, dans laquelle l'ensemble de circuits de traitement du dispositif de commande (202) sont configurés pour déterminer si la pompe piézoélectrique (116) n'est pas opérationnelle sur la base de la fréquence d'oscillation.

8. Unité de thérapie selon la revendication 5, comprenant en outre un capteur de pression (118) accouplé fluidiquement au site de plaie et configuré pour lire une pression au niveau du site de plaie, dans laquelle l'ensemble de circuits de traitement du dispositif de commande (202) est configuré pour faire fonctionner la première pompe (112) pour aspirer la pression négative au niveau du site de plaie sur la base de la pression au niveau du site de plaie.

9. Unité de thérapie selon la revendication 8, dans laquelle le dispositif de commande est (202) configuré pour faire fonctionner la première pompe (112) et la pompe piézoélectrique (116) simultanément.

10. Unité de thérapie selon la revendication 8, dans laquelle la première pompe (112) est une pompe à membrane.

11. Unité de thérapie selon la revendication 1, comprenant en outre un boîtier (106), dans laquelle la pompe à membrane (112) et la pompe piézo-électrique (116) sont positionnées à l'intérieur du boîtier (106).

12. Unité de thérapie selon la revendication 1, comprenant en outre une cartouche (108) conçue pour collecter un fluide aspiré du site de la plaie en raison du fonctionnement de la première pompe (112).

13. Système de thérapie de plaie par pression négative comprenant une unité de thérapie selon l'une quelconque des revendications 1 à 12.
